# FASCICULE DE BREVET EUROPEEN

(11) **EP 2 929 390 B1**
(45) Date de publication et mention de la délivrance du brevet: **28.02.2018**
(21) Numéro de dépôt: 13812006.8
(22) Date de dépôt: 27.11.2013
(51) Int. Cl.: G02B 27/00, G02B 27/01, A61B 3/00, A61F 9/00, G09B 21/00, H04N 7/18

(54) **DISPOSITIF PERMETTANT D'AMELIORER LA VISION D'UN OEIL HUMAIN**
VORRICHTUNG ZUR VERBESSERUNG DES MENSCHLICHEN AUGES
DEVICE FOR IMPROVING VISION OF A HUMAN EYE

(30) Priorité: 10.12.2012 FR 1203345
(43) Date de publication de la demande: 14.10.2015
(73) Titulaire: Light Vision, 92400 Courbevoie (FR)
(72) Inventeur: AIT-YAHIATENE, Daniel, F-92150 Suresnes (FR)
(74) Mandataire: Flavenot, Bernard
(86) Numéro de dépôt international: PCT/FR2013/000310
(87) Numéro de publication internationale: WO 2014/091091

(56) Documents cités:
- EP-A2- 0 216 692
- FR-A1- 2 766 931
- FR-A1- 2 964 755

## Description

La présente invention concerne les dispositifs permettant d'améliorer la vision d'un être humain, qui trouvent une application particulièrement avantageuse pour améliorer la vision d'un être humain dont au moins un oeil est atteint de DMLA (Dégénérescence Maculaire Liée à l'Age), mais aussi de nombreuses autres applications par exemple industrielles, militaires et civiles.

La DMLA est une maladie de la rétine qui peut être définie de la façon suivante : "Ensemble des lésions de la région maculaire, dégénératives, non inflammatoires, acquises, survenant sur un oeil auparavant normal, apparaissant après l'âge de cinquante ans et entraînant une altération de la fonction maculaire et de la vision centrale".

Les causes précises de cette maladie ne sont pas connues et on ne sait pas encore actuellement la guérir. Les traitements existants permettent seulement de ralentir son évolution.

Aussi, pour compenser cette perte de vision, ont été mis au point des dispositifs pour améliorer la vision d'un oeil atteint de DMLA, par exemple les dispositifs décrits dans la publication FR 2 964 755 A1 et accessoirement dans les publications FR 2 766 931 A1 et EP 0 216 592 A2. Plus particulièrement, le dispositif selon la publication FR 2 964 755 A1 permet d'améliorer la vision d'un oeil d'un être humain, l'oeil pivotant dans son orbite et comportant un cristallin et une rétine dont une zone est atteinte de DMLA (Dégénérescence Maculaire Liée à l'Age). Le dispositif décrit dans ce brevet donne de bons résultats mais présente des inconvénients, notamment par le fait qu'il comporte des éléments/pièces en mouvement qui, d'une façon générale, sont évidemment moins fiables que des éléments statiques, et qui sont en plus consommateurs d'énergie, ce qui implique l'utilisation de sources d'énergie qui sont relativement encombrantes et qui demandent à être fréquemment rechargées. En se référant plus particulièrement à la figure 2 de ce document, tel est par exemple le cas du déflecteur désigné sous la référence 50 qui est constitué par un ensemble d'éléments qui doivent être en mouvement pour donner le résultat recherché, par exemple les miroirs sur des rails associés aux moteurs qui les commandent.

Aussi, la présente invention a-t-elle pour but de réaliser un dispositif pour améliorer la vision d'un oeil d'un être humain, qui palie en grande partie les inconvénients des dispositifs de l'art antérieur, quelles que soient les applications prévues, qui ne comporte pas d'éléments en mouvement et qui soit de ce fait plus simple et plus fiable que les dispositifs similaires de l'art antérieur.

Plus précisément, la présente invention a pour objet un dispositif pour améliorer la vision d'un oeil d'un être humain, ledit oeil pivotant dans son orbite et comportant un cristallin et une rétine, comme plus particulièrement défini dans la revendication annexée.

D'autres caractéristiques et avantages de la présente invention apparaîtront au cours de la description suivante donnée en regard du dessin annexés à titre illustratif mais nullement limitatif, dans lesquels :
La figure 1 est un schéma bloc de principe d'un mode de réalisation du dispositif selon l'invention pour permettre une meilleure vision d'un oeil d'un être humain dans le cas où il est atteint de DMLA, et
Les figures 2 et 3 représentent, sous forme très schématique, une partie du dispositif selon la figure 1, dans deux états différents pour expliciter son fonctionnement.

Il est aussi précisé que les figures représentent un mode de réalisation de l'objet selon l'invention, mais qu'il peut exister d'autres modes de réalisation qui répondent à la définition de cette invention.

Il est aussi précisé que, dans la présente description, si l'adverbe "sensiblement" est associé à un qualificatif d'un moyen donné, ce qualificatif doit être compris au sens strict ou approché.

La présente invention concerne un dispositif pour améliorer la vision d'un oeil 1 d'un être humain, l'oeil pivotant dans son orbite 2 et comportant un cristallin 7 et une rétine 8.

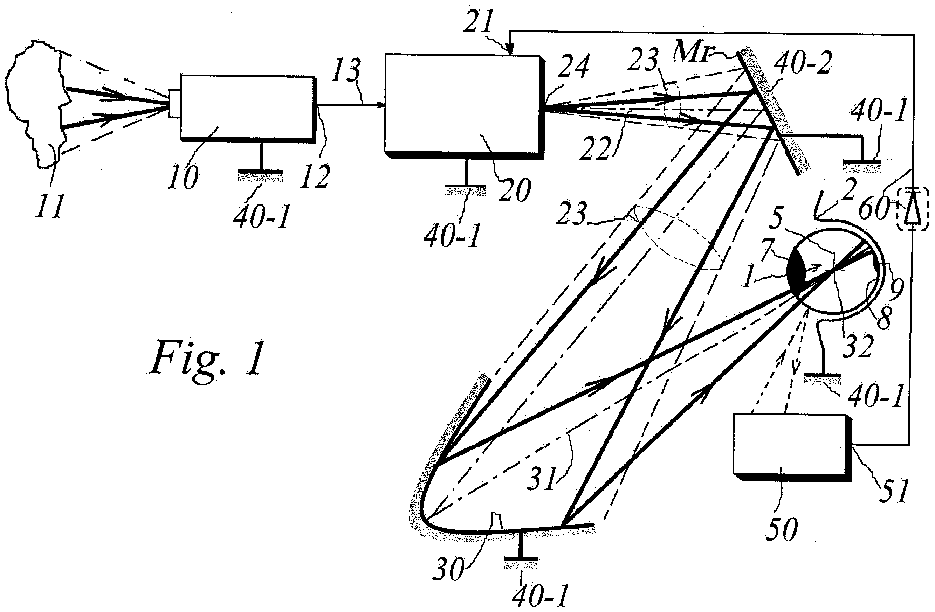

Le dispositif, en référence plus particulièrement à la figure 1, comporte des moyens optiques 10 pour former au moins une image d'une scène à visualiser 11, un projecteur optique de traitement commandable 20, par exemple par microprocesseur ou analogue, monté en coopération 13, 14 avec les moyens optiques 10, le projecteur étant apte (i) à émettre en sortie 24 l'image sous la forme d'un faisceau lumineux image 22, et (ii) à être commandé par un signal de valeurs déterminées appliqué à une entrée de commande 21 de façon que le faisceau lumineux image 22 soit dévié suivant un angle de déflexion fonction de la valeur du signal.

Un tel projecteur optique de traitement commandable 20 peut prendre différentes formes, selon la nature de l'image délivrée en sortie 24 des moyens optiques 10 qui peuvent du type électronique ou même purement optique.

Dans une réalisation particulièrement avantageuse, quand il est connecté en sortie d'une caméra vidéo ou analogue qui délivre une image sous forme de signaux informatiques numériques, ce projecteur optique 20 est un projecteur comme celui qui est commercialisé par la Société "Texas Instruments" sous la Marque de commerce "*DMD Discovery*™ *3000*"*.*

Le dispositif comporte en outre un miroir 30, et des moyens 40-1, 40-2 pour lier ce miroir 30 à l'orbite 2 et au projecteur optique de traitement 20 de façon que l'axe optique 31 du miroir 30 passe sensiblement constamment par le centre de rotation 5 de l'oeil 1 et que ce miroir 30 soit sur le trajet optique du faisceau lumineux image 22 défini ci-dessus.

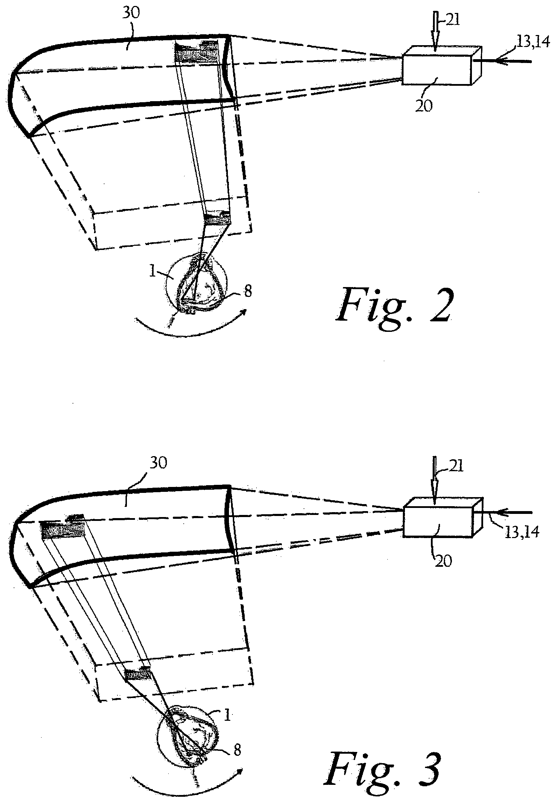

Ces moyens de liaison 40-1, 40-2 schématiquement illustrés sur la figure 1 seront par exemple très préférentiellement du type de ceux décrits dans la publication FR 2 964 755 A1 mentionnée ci-avant au nom du Demandeur et illustrés sur la figure 3 de ce document. Ils seront désignés ici, dans la présente description, sous le terme générique de "lunettes", du fait de leur ressemblance avec cet objet de vue bien connu.

Il est précisé que le miroir de renvoi référencé Mr n'est représenté, sur la figure 1, que comme une possibilité et qu'il n'est pas obligatoirement nécessaire. Il a été prévu sur la figure 1, uniquement pour la commodité de la représentation du dessin afin de dilater les éléments constitutifs du dispositif. C'est ainsi qu'il sera possible que, du fait d'une miniaturisation, le dispositif finalement réalisé n'ait pas besoin de ce miroir de renvoi Mr.

Il est en outre prévu, un capteur 50 pour déterminer la rotation de l'oeil 1 par rapport à son orbite 2, ce capteur étant apte à délivrer en sortie 51 un signal représentatif de cette rotation, et des moyens 60 pour appliquer le signal délivré en sortie 51 du capteur 50 à l'entrée 21 du projecteur optique de traitement 20 de façon que le faisceau lumineux image 22 soit projeté sur un endroit du miroir 30 fonction de la rotation de l'oeil 1 dans son orbite 2, de façon que l'image, réfléchie sur le miroir se crée directement sur la rétine de l'oeil, comme schématiquement illustré sur les figures 2 et 3.

Selon une réalisation particulièrement avantageuse qui permet d'attendre les buts définis ci-avant, le miroir 30 a la forme sensiblement d'un paraboloïde de révolution dont le point focal 32 est sensiblement confondu avec le centre de rotation 5 de l'oeil 1 dans son orbite 2.

Un tel miroir peut avoir différentes structures, par exemple celle d'un miroir optique avec une surface réfléchissante présentant la forme d'un paraboloïde de révolution. Cependant, de façon préférentielle, du fait notamment de son encombrement très réduit par rapport à la réalisation évoquée ci-dessus, le miroir 30 peut être un miroir holographique, donc réalisé sur une surface plane. Un tel miroir est bien connu en lui-même et des hommes du métier.

Comme illustré sur la figure 1, le miroir 30 est positionné, par rapport à l'orbite 2 de l'oeil 1, de façon que le faisceau lumineux image 22 soit réfléchi par ce miroir 30, puis focalisé par le cristallin 7 sur la rétine 8, en un endroit non situé dans la zone 9 dans le cas de l'utilisation du dispositif pour améliorer la vision d'un oeil atteint de DMLA.

Les hommes du métier n'auront aucune difficulté pour réaliser le dispositif selon l'invention tel que décrit ci-dessus, notamment en se référant au contenu du brevet du Demandeur référencé ci-avant.

Quant à son fonctionnement, il se déduit sans difficulté de la présente description et des deux figures 2 et 3 annexées.

Il est cependant précisé que l'image générée par le projecteur 20 peut avoir été prise dans un champ supérieur au champ de vision de l'oeil. Seule, une fenêtre correspondant à ce champ visuel sera alors ouverte et, dans cette fenêtre, il sera généré une image prise par la caméra située sur, par exemple, des lunettes (voir la publication FR 2 964 755 A1) adaptées au traitement adaptées au traitement correspondant au besoin visuel de l'oeil 1.

S'il le souhaite, l'utilisateur pourra agir sur le zoom de la caméra pour focaliser sur un objet et/ou une partie de paysage. L'image envoyée sera adaptée à son meilleur angle de vision formé entre la vision centrale représentée par le centre de la pupille et la vision périphérique de la rétine encore exploitable.

Un calibrage automatique sera réalisé lors de la mise en route du dispositif. L'angle obtenu par ce calibrage sera enregistré dans la mémoire du microprocesseur de pilotage du projecteur 20 défini ci-avant et utilisé lors de la projection.

Comme mentionné au préambule de la présente description, le dispositif peut trouver une application très intéressante pour l'amélioration de la vision d'un être humain dont la rétine 8 d'au moins un l'oeil présente une zone 9 atteinte de DMLA (Dégénérescence Maculaire Liée à l'Age).

Mais il peut trouver des applications dans de nombreux autres domaines industriels, militaires et civils lorsqu'il est nécessaire d'avoir une vision améliorée, par exemple du type « réalité augmentée » pour des applications de maintenance dans l'industrie automobile, aéronautique, de recherche sur des étagères de stockage, etc., pour aider les chirurgiens en leur donnant des informations sur les organes non visibles en direct et utiles à l'intervention, pour mieux informer des personnes dans le domaine de la navigation pédestre, routière, aérienne, etc., pour rechercher des lieux par superposition d'images aux images réelles vues par l'utilisateur, etc.

Les descriptions structurelle et de fonctionnement données ci-dessus mettent en évidence le fait que les buts de l'invention ont été atteints, notamment le fait qu'il n'y a plus, dans le dispositif, de pièces/éléments importants en mouvement, ce qui permet de mieux le miniaturiser et ainsi l'intégrer plus aisément sur un élément de type « lunettes ».

## Revendications

1. Dispositif pour améliorer la vision d'un oeil (1) d'un être humain, ledit oeil pivotant dans son orbite (2) et comportant un cristallin (7) et une rétine (8), comportant :
• des moyens optiques (10) pour former au moins une image d'une scène à visualiser (11),
• un projecteur optique de traitement commandable (20) monté en coopération (13, 14) avec lesdits moyens optiques (10), ledit projecteur étant apte à émettre en sortie (24) ladite image sous la forme d'un faisceau lumineux image (22), et à être commandé par un signal de valeurs déterminées appliqué à une entrée de commande (21) de façon que le faisceau lumineux image (22) soit dévié suivant un angle de déflexion fonction de la valeur du dit signal,
• un miroir (30),
• des moyens (40-1, 40-2) pour lier ledit miroir (30) à l'orbite (2) et au projecteur optique de traitement (20) de façon que l'axe optique (31) du miroir (30) passe sensiblement par le centre de rotation (5) du dit oeil (1) et que ce miroir (30) soit sur le trajet optique du faisceau lumineux image (22), et
• un capteur (50) pour déterminer la rotation du dit oeil (1) par rapport à son orbite (2), ce capteur étant apte à délivrer en sortie (51) un signal représentatif de cette rotation,
**caractérisé par le fait qu'**il comporte en outre des moyens (60) pour appliquer ledit signal délivré en sortie (51) du capteur (50) à l'entrée (21) du projecteur optique de traitement (20) de façon que le faisceau lumineux image (22) soit projeté sur un endroit du dit miroir (30) fonction de la rotation du dit oeil (1) par rapport à son orbite (2).

2. Dispositif selon la revendication 1, **caractérisé par le fait que** ledit miroir (30) a la forme sensiblement d'un paraboloïde de révolution dont le point focal (32) est sensiblement confondu avec le centre de rotation (5) de l'oeil (1) dans son orbite (2).

3. Dispositif selon l'une des revendications 1 et 2, **caractérisé par le fait que** ledit miroir (30) est un miroir holographique.

4. Dispositif selon l'une des revendications 1 à 3, **caractérisé par le fait que** le projecteur optique (20) est un projecteur comme celui qui est commercialisé par la Société "Texas Instruments" sous la Marque de commerce "*DMD Discovery*™ *3000*"*.*

5. Dispositif selon l'une des revendications précédentes pour améliorer la vision d'un oeil (1) d'un être humain dont une zone (9) de la rétine (8) est atteinte de DMLA (Dégénérescence Maculaire Liée à l'Age), **caractérisé par le fait que** ledit miroir (30) est positionné, par rapport à l'orbite (2), de façon que le faisceau lumineux image (22) soit réfléchi par ce miroir (30), puis focalisé par le cristallin (7) sur la rétine (8) en un endroit non situé dans ladite zone (9).

## Patentansprüche

1. Vorrichtung zum Verbessern des Sehvermögens eines menschlichen Auges (1), wobei das Auge in seiner Augenhöhle (2) schwenkt und eine Linse (7) und eine Retina (8) besitzt, umfassend:
- optische Mittel (10) zum Erzeugen mindestens eines Bildes einer anzuzeigenden Szene (11),
- einen optischen Projektor (20) mit steuerbarer Verarbeitung, der in Zusammenwirkung (13, 14) mit den optischen Mitteln (10) montiert ist, wobei der Projektor fähig ist, an seinem Ausgang (24) das Bild in Form eines Bild-Lichtstrahlenbündels (22) auszusenden und durch ein Signal mit bestimmten Werten gesteuert zu werden, das in einen Steuereingang (21) eingegeben wird, derart, dass das Bild-Lichtstrahlenbündel (22) entsprechend einem Ablenkwinkel als Funktion des Wertes des Signals abgelenkt wird,
- einen Spiegel (30),
- Mittel (40-1, 40-2), um den Spiegel (30) mit der Augenhöhle (2) und mit dem optischen Verarbeitungsprojektor (20) in einer Weise zu verbinden, dass die optische Achse (31) des Spiegels (30) im Wesentlichen durch das Drehzentrum (5) des Auges (1) verläuft und dass sich dieser Spiegel (30) auf dem optischen Weg des Bild-Lichtstrahlenbündels (22) befindet, und
- einen Sensor (50) zum Bestimmen der Drehung des Auges (1) in Bezug auf seine Augenhöhle (2), wobei dieser Sensor fähig ist, an seinem Ausgang (51) ein diese Drehung darstellendes Signal auszugeben,
**dadurch gekennzeichnet, dass** sie ferner Mittel (60) umfasst, um das am Ausgang (51) des Sensors (50) ausgegebene Signal in den Eingang (21) des optischen Verarbeitungsprojektors (20) einzugeben, derart, dass das Bild-Lichtstrahlenbündel (22) als Funktion der Drehung des Auges (1) in Bezug auf seine Augenhöhle (2) auf eine Stelle des Spiegels (30) projiziert wird.

2. Vorrichtung nach Anspruch 1, **dadurch gekennzeichnet, dass** der Spiegel (30) im Wesentlichen die Form eines rotationssymmetrischen Paraboloids hat, dessen Brennpunkt (32) im Wesentlichen mit dem Drehzentrum (5) des Auges (1) in seiner Augenhöhle (2) zusammenfällt.

3. Vorrichtung nach einem der Ansprüche 1 und 2, **dadurch gekennzeichnet, dass** der Spiegel (30) ein holographischer Spiegel ist.

4. Vorrichtung nach einem der Ansprüche 1 bis 3, **dadurch gekennzeichnet, dass** der optische Projektor (20) ein Projektor wie jener ist, der von der Firma "Texas Instruments" unter der Handelsmarke "*DMD Discovery*™ *3000*" vermarktet wird.

5. Vorrichtung nach einem der vorhergehenden Ansprüche, um das Sehvermögen eines menschlichen Auges (1) zu verbessern, wovon ein Bereich (9) der Retina (8) eine AMD (Altersbedingte Makuladegeneration) erreicht hat, **dadurch gekennzeichnet, dass** der Spiegel (30) in Bezug auf die Augenhöhle (2) in einer Weise positioniert ist, dass das Bild-Lichtstrahlenbündel (22) durch diesen Spiegel (30) reflektiert und dann durch die Linse (7) auf die Retina (8) an einer Stelle, die sich nicht in diesem Bereich (9) befindet, fokussiert wird.

## Claims

1. Device for improving the vision of an eye (1) of a human being, said eye pivoting in its orbit (2) and including a lens (7) and a retina (8), comprising:
• optical means (10) for forming at least one image of a scene to be viewed (11);
• a controllable treatment optical projector (20) mounted to co-operate (13, 14) with said optical means (10), said projector being suitable for outputting (24) said image in the form of an image light beam (22), and for being controlled by a signal of determined values applied to a control input (21) so that the image light beam (22) is deflected through a deflection angle that is a function of the value of said signal;
• a mirror (30);
• means (40-1, 40-2) for linking said mirror (30) with the orbit (2) and with the treatment optical projector (20) so that the optical axis (31) of the mirror (30) passes substantially through the center of rotation (5) of said eye (1), and so that the mirror (30) lies on the light path of the image light beam (22); and
• a sensor (50) for determining the rotation of said eye (1) relative to its orbit (2), the sensor being suitable for outputting (51) a signal representative of the rotation;
**characterized by** the fact that it further comprises means (60) for applying said signal delivered at the output (51) of the sensor (50) to the input (21) of the treatment optical projector (20) so that the image light beam (22) is projected onto a location of said mirror (30) that is a function of the rotation of said eye (1) relative to its orbit (2).

2. Device according to claim 1, **characterized by** the fact that said mirror (30) is substantially in the form of a paraboloid of revolution having its focal point (32) coinciding substantially with the center of rotation (5) of the eye (1) in its orbit (2).

3. Device according any one of the claim 1 and 2, **characterized by** the fact that said mirror (30) is a holographic mirror.

4. Device according to any one of claims 1 to 3, **characterized by** the fact that the optical projector (20) is a projector such as that sold by the supplier "Texas Instruments" under the trademark "DMD Discovery™ 3000".

5. Device according to any preceding claim for improving the vision of an eye (1) of a human being, the eye having a zone (9) of its retina (8) suffering from age-related macular degeneration (ARMD), the device being **characterized by** the fact that said mirror (30) is positioned relative to the orbit (2) in such a manner that the image light beam (22) is reflected by the mirror (30) and then focused by the lens (7) onto the retina (8) at a location that is not situated in said zone (9).
